# FASCICULE DE BREVET EUROPEEN

(11) **EP 1 207 891 B1**
(45) Date de publication et mention de la délivrance du brevet: **09.07.2008**
(21) Numéro de dépôt: 00960777.1
(22) Date de dépôt: 01.09.2000
(51) Int. Cl.: A61K 31/737, A61P 7/02

(54) **UTILISATION D'UN POLYSACCHARIDE SULFATE DE BAS POIDS MOLECULAIRE POUR LE TRAITEMENT DES THROMBOSES**
VERWENDUNG DER SULFATIERTEN POLYSACCHARIDEN ZUR BEHANDLUNG VON THROMBOSEN
USE OF A LOW MOLECULAR WEIGHT SULPHATED POLYSACCHARIDE IN THE TREATMENT OF THROMBOSIS

(30) Priorité: 01.09.1999 FR 9910965
(43) Date de publication de la demande: 29.05.2002
(73) Titulaire: INSTITUT FRANCAIS DE RECHERCHE POUR L'EXPLOITATION DE LA MER (IFREMER), 92138 Issy-les-Moulineaux Cedex (FR); CENTRE NATIONAL DE LA RECHERCHE SCIENTIFIQUE (CNRS), 75794 Paris Cedex 16 (FR); UNIVERSITE RENE DESCARTES, (PARIS V), F-75006 Paris (FR)
(72) Inventeur: COLLIEC-JOUAULT, Sylvia, F-44300 Nantes (FR); DURAND, Patrick, F-44400 Rèze (FR); FISCHER, Anne-Marie, F-75013 Paris (FR); JOZEFONVICZ, Jacqueline, F-60260 Lamorlaye (FR); LETOURNEUR, Didier, F-92350 Le Plessis Robinson (FR); MILLET, Jean, F-21910 Corcelles Les Citeaux (FR)
(74) Mandataire: Nargolwalla, Cyra
(86) Numéro de dépôt international: PCT/FR2000/002421
(87) Numéro de publication internationale: WO 2001/015654

(56) Documents cités:
- EP-A- 0 403 377
- EP-A- 0 408 770
- EP-A- 0 849 280
- WO-A-97/08206
- MILLET: "Antithrombotic and anticoagulant activities of a low molecular weight fucoidan by the subcutaneous route" THROMB. HAEMOSTAS., vol. 81, no. 3, mars 1999 (1999-03), pages 391-395, XP000921119
- MCCAFFREY T A ET AL: "FUCOIDAN IS A NON-ANTICOAGULANT INHIBITOR OF INTIMAL HYPERPLASTA" BIOCHEMICAL AND BIOPHYSICAL RESEARCH COMMUNICATIONS,US,ACADEMIC PRESS INC. ORLANDO, FL, vol. 184, no. 2, 1 janvier 1992 (1992-01-01), pages 773-781, XP002061174 ISSN: 0006-291X
- MAURAY S ET AL: "VENOUS ANTITHROMBOTIC AND ANTICOAGULANT ACTIVITIES OF A FUCOIDAN FRACTION" THROMBOSIS AND HAEMOSTASIS,DE,STUTTGART, vol. 74, no. 5, 1 novembre 1995 (1995-11-01), pages 1280-1285, XP000671773 ISSN: 0340-6245 cité dans la demande
- GRAUFFEL V ET AL: "NEW NATURAL POLYSACCHARIDES WITH POTENT ANTITHROMBIC ACTIVITY: FUCANS FROM BROWN ALGAE" BIOMATERIALS,GB,ELSEVIER SCIENCE PUBLISHERS BV., BARKING, vol. 10, no. 6, 1 août 1989 (1989-08-01), pages 363-368, XP000050302 ISSN: 0142-9612
- LOGEART ET AL.: "Fucans, sulfated polysaccharides extracted from brown seaweeds, inhibit vascular smooth muscle cell proliferation. I. Comparison with heparin for antiproliferative activity, binding and internalisation" EUR. J. CELL. BIOL. (2 ARTICLES DU MÊME AUTEUR), vol. 74, décembre 1997 (1997-12), pages 376-390, XP000921114 cité dans la demande
- COLLIEC ET AL.: "A low molecular weight fucoidan fraction from the brown seaweed Pelvetia canaliculata" PHYTOCHEMISTRY, vol. 35, no. 3, 1994, pages 697-700, XP000921147

## Description

La présente invention a pour objet l'utilisation d'un polysaccharide sulfaté de masse molaire inférieure ou égale à 10 000 g/mol, susceptible d'être obtenu par dépolymérisation radicalaire d'un fucane brut issu de Phéophycées, pour l'obtention d'un médicament actif contre la thrombose artérielle et contre la resténose artérielle.

La thrombose consiste en la formation d'un caillot (thrombus) dans l'appareil circulatoire, caillot qui obstrue la lumière du vaisseau dans lequel il se forme. Elle est la conséquence de l'activation pathologique des phénomènes physiologiques de l'hémostase, c'est-à-dire des phénomènes qui concourent à la prévention et à l'arrêt des saignements.

La thrombose met en jeu un processus complexe impliquant l'activation en cascade de différents facteurs, aboutissant à la formation de la thrombine, enzyme clé de la coagulation, puis à la fibrinoformation. La formation du thrombus débute par l'adhésion des plaquettes au tissu conjonctif sous-endothélial, mis à nu par une lésion de l'endothélium vasculaire. Les plaquettes s'agrègent entre elles et l'agrégat s'entoure d'un réseau de fibrine, qui emprisonne également des globules blancs et des globules rouges, formant le thrombus.

La thrombose artérielle diffère de la thrombose veineuse en ce qu'elle survient le plus_ souvent sur une artère lésée par la présence d'une plaque d'athérome ; cette lésion se caractérise en outre par la prolifération et la migration vers l'intima des cellules musculaires lisses de la *media.* La thrombose artérielle se produit souvent lors de la rupture de la plaque d'athérome, avec perte de continuité de l'endothélium vasculaire. L'adhésion et l'agrégation des plaquettes jouent un rôle primordial dans le phénomène de thrombose artérielle.

L'ensemble du processus de réponse à la lésion d'une artère implique de nombreux phénomènes biologiques cellulaires mettant en jeu des modifications du phénotype des cellules musculaires lisses (CML), ainsi que l'expression de facteurs de croissance qui favorisent la prolifération des cellules endothéliales.

Dans le traitement de la thrombose veineuse, on utilise classiquement des anticoagulants, notamment l'héparine, qui possèdent la propriété d'inhiber la thrombine et sa formation.

L'héparine est un polysaccharide sulfaté constitué d'unités de glucosamine et d'acides uroniques liées en 1-4, dans lesquelles les groupes sulfates sont présents sur la fonction amine de la glucosamine et/ou sur des fonctions alcools de la glucosamine et de l'acide uronique. Ce polysaccharide, dont les propriétés anticoagulantes sont bien connues, est actuellement largement utilisé dans le traitement des accidents thrombotiques. L'héparine présente toutefois des effets secondaires très importants (saignements, risques de thrombopénies immuno-allergiques) et elle est très peu efficace en thrombose artérielle. De plus, l'origine animale de ce produit est susceptible d'entraîner un risque potentiel de contamination par des agents infectieux non conventionnels.

Des techniques de dépolymérisation chimique ou enzymatique ont permis d'obtenir, à partir d'HNF (héparine non fractionnée, dont le poids moléculaire est d'environ 15 000 g/mol), des chaînes polysaccharidiques de bas poids moléculaire, à savoir de poids moléculaire compris entre 2 000 et 10 000 g/mol, nommées HBPM (Héparines de Bas Poids Moléculaire). De nombreuses HBPM ont été synthétisées et sont notamment commercialisées sous les dénominations Enoxaparine^{®}, Reviparine^{®}, Dalteparine^{®}, Fraxiparine^{®}, Tinzaparine^{®}, Certoparine^{®}, Opocrine^{®}, Parnaparine^{®}, etc.

Des études cliniques ont montré que, dans la prophylaxie des accidents thrombo-emboliques veineux, les HBPM possèdent une efficacité identique, sinon supérieure, à celle de l'HNF. Toutefois, les HBPM n'abolisent pas le risque hémorragique et peuvent entraîner, au même titre que l'HNF, quoique avec une moindre fréquence, une thrombopénie immuno-allergique.

D'autre part, il a été montré, en particulier par M. Lerch et al. (European Heart Journal, Août 1998, 19, 495) et H. Rickli et al. (European Heart Journal, Août 1998, 19, 470), que des HBPM (respectivement, Reviparin^{®} et Fraxiparin^{®}) sont inefficaces pour lutter contre la resténose après angioplastie, c'est-à-dire le phénomène de réapparition d'un rétrécissement de la lumière d'une artère lié à l'intervention d'une sonde à ballonnet en -chirurgie vasculaire.

Il existe d'autres polysaccharides sulfatés que les héparines, par exemple les fucanes. Ces polysaccharides sulfatés, de poids moléculaire élevé (100 à 800 kDa), sont présents dans les parois cellulaires des thalles d'algues brunes. Il s'agit de polymères de L-fucose sulfaté qui peuvent également contenir du D-xylose, du D-galactose, du D-mannose et des acides uroniques, ces derniers n'étant pas sulfatés, contrairement à ceux de l'héparine. Les fucanes diffèrent également de l'héparine en ce qu'ils ne comprennent pas d'amino-sucres.

Les fucanes possèdent différentes propriétés qui rendent particulièrement intéressante leur exploitation dans de nombreux domaines thérapeutiques.

Il a notamment été montré que des fractions de fucane de faible masse molaire, obtenues par hydrolyse acide tel que décrit dans le Brevet européen 0 403 377, présentent une activité anticoagulante (S. Colliec et al., Thromb. Res., 1991, 64, 143-154) et antithrombotique par voie intraveineuse (S. Mauray et al., Thrombosis and Haemostasis, 1995, 74(5), 1280-1285) et par voie sous-cutanée (J. Millet et al., Thrombosis and Haemostasis, 1999, 81, 391-395) comparable à celle des héparines de bas poids moléculaires.

Il a également été montré que ces mêmes fractions de fucane sont capables d'inhiber, comme l'héparine, la croissance des cellules musculaires lisses vasculaires en culture (D. Logeart et al., Eur. J. Cell. Biol., 1997, 74, 376-384 et 385-390). Les effets observés sont réversibles, ne sont pas liés à une action cytotoxique et dépendent de la concentration du composé dans le milieu de culture. Cet effet antiprolifératif sur la croissance des cellules musculaires lisses semble spécifique puisqu'il n'est noté, à ces concentrations, aucune inhibition sur la croissance de lignées fibroblastiques, et que ces composés sont capables de potentialiser la croissance de cellules endothéliales en culture (J. L. Giraux et al., Eur. J. Cell. Biol, 1998, 77, 352-359).

Giraux et al. ont montré, dans Thromb. Haemost., 1998, 80, 692-695, que ces mêmes fractions de fucane, obtenues par hydrolyse acide selon le protocole décrit dans le Brevet européen 0 403 377, induisent *in vitro* la libération de TFPI (Tissue Factor- Pathway Inhibitor) par les cellules endothéliales de la veine de cordon ombilical humain, effet qui pourrait contribuer à l'action antithrombotique de ces fractions de fucane.

D'autre part, il a été montré dans la Demande de Brevet EP 0 846 129 que des fragments de fucane, obtenus par dépolymérisation radicalaire d'un fucane de Phéophycées en présence d'un catalyseur métallique et de peroxyde d'hydrogène et possédant une masse molaire inférieure ou égale à 10 000 g/mol, conservent, *in vitro,* les propriétés anticoagulantes du fucane brut. De tels fragments de fucane, obtenus par dépolymérisation radicalaire d'un fucane de haut poids moléculaire, sont différentes, quant à leur structure chimique, de fragments de fucane obtenus par hydrolyse acide d'un fucane brut, comme démontré dans la Demande de Brevet EP 0 846 129.

Outre l'HNF, les HBPM et les fucanes, d'autres agents anticoagulants ont été décrits pour leur action antithrombotique (inhibition de la formation du thrombus et/ou de sa croissance) : il s'agit notamment des héparinoïdes (mélange de glycosaminoglycanes de bas poids moléculaires, par exemple l'Orgaran^{®} commercialisé par Organon Inc.), des antivitamines K et des hirudines. Les hirudines, par exemple la Lepirudin (Refludan^{®}) commercialisée par Behrinwerke AG - Hoechst Marion Roussel ou la Desirudin (Revasc^{®}) commercialisée par Novartis - Rhône Poulenc Rorer, peuvent entraîner, au même titre que l'héparine non fractionnée, un risque hémorragique important.

En thrombose artérielle, deux classes de composés sont utilisables ou à l'étude (Samama M. M. et Desnoyer P. C., « Les bases pharmacologiques des traitements antithrombotiques - Agents antithrombotiques actuels et futurs », 1995, Edition Masson) : il s'agit des antiagrégants plaquettaires , par exemple l'acide acétylsalicylique, le dipyridamole, la ticlopidine, le clopidogrel, l'anticorps anti-GPIIb/IIIa, et des fibrinolytiques (streptokinase, urokinase, etc), qui dissolvent le caillot par activation du système fibrinolytique et libération de la plasmine (enzyme protéolytique capable de lyser rapidement le caillot de fibrine).

L'utilisation de ces composés doit souvent être associée à une intervention endoscopique pour élargir la lumière de l'artère rétrécie, telle qu'une angioplastie. Or, ces traitements ne sont pas suffisamment efficaces à moyen terme, car une resténose des artères ainsi désobstruées survient fréquemment, et ce dans les quelques mois qui suivent le traitement.

Ainsi, il apparaît que les agents antithrombotiques décrits ci-dessus ne permettent pas de prévenir ou de traiter de façon efficace une thrombose artérielle ou une resténose, problème essentiel après une angioplastie. Suite à une telle chirurgie, l'endothélium présente un disfonctionnement par rapport à l'endothélium d'origine. L'agression pariétale induite par cette chirurgie peut se traduire par un phénomène - de décompartimentation : perte de l'endothélium et communications directes entre le compartiment sanguin et les cellules musculaires lisses. A cette agression, la paroi répond par un processus associant une migration, une prolifération, une destruction et une sécrétion de matrice extracellulaire par les cellules musculaires lisses en position intimale, suivi d'une repousse de l'endothélium, ce qui contribue à son épaississement et donc au rétrécissement de sa lumière. Ce phénomène de cicatrisation pariétale peut participer à la pathologie elle-même, comme les sténoses anastomotiques sur les prothèses vasculaires et les resténoses après dilatation endoluminale.

Au vu de l'état actuel de la technique, le besoin d'un agent actif contre la thrombose et la resténose artérielles se fait donc ressentir.

Les Inventeurs se sont ainsi donnés pour but de pourvoir à un agent qui puisse être utilisé pour l'obtention d'un médicament actif contre la thrombose et la resténose artérielles, ledit médicament :
- présentant une activité contre la thrombose vasculaire, notamment contre la thrombose veineuse, et de façon particulièrement avantageuse contre la thrombose artérielle, et ce lorsqu'il est administré par voie parentérale,
- ne présentant pas de risque hémorragique majeur,
- ne présentant pas le risque potentiel de contamination virale lié aux produits d'origine animale,
- permettant d'obtenir une prévention de l'occlusion artérielle dans le cadre de la prévention de la resténose, en particulier à la suite d'une angioplastie.

Les Inventeurs ont maintenant trouvé que, de manière inattendue, ces buts sont atteints par l'utilisation d'un polysaccharide sulfaté susceptible d'être obtenu par dépolymérisation radicalaire d'un fucane brut issu de Phéophycées, ledit polysaccharide ayant une masse molaire inférieure ou égale à 10 000 g/mol.

La présente invention a pour objet l'utilisation d'un polysaccharide sulfaté susceptible d'être obtenu par dépolymérisation radicalaire d'un fucane brut issu de Phéophycées, ledit polysaccharide ayant une masse molaire inférieure ou égale à 10 000 g/mol, pour l'obtention d'un médicament destiné à la prévention ou au traitement de la thrombose vasculaire.

De façon particulièrement avantageuse, l'utilisation d'un tel polysaccharide sulfaté permet d'obtenir un effet antithrombotique qui n'est pas accompagné d'un risque hémorragique majeur.

Ledit polysaccharide sulfaté est susceptible d'être obtenu comme décrit dans la Demande de Brevet EP 0 846 129. A titre d'exemple, le type de Phéophycées dont est issu ledit polysaccharide sulfaté est *Ascophyllum nodosum, Fucus vesiculosus, Pelvetia canaliculata* ou *Undaria pinnatifida.*

De façon particulièrement avantageuse, l'origine végétale du polysaccharide utilisé dans la présente invention élimine tout risque de contamination virale du sujet auquel il est administré.

Selon un mode de réalisation avantageux de l'utilisation selon l'invention, ledit médicament est destiné à la prévention ou au traitement de la thrombose veineuse.

Selon un autre mode de réalisation avantageux de l'utilisation selon l'invention, ledit médicament est destiné à la prévention ou au traitement de la thrombose artérielle, processus dans lequel la formation du thrombus et les dépôts plaquettaires jouent un rôle important.

Selon une disposition avantageuse de ce mode de réalisation, ledit médicament est destiné à la prévention de la resténose artérielle, phénomène précurseur d'une thrombose artérielle.

En effet, dans le cadre de la prévention de la thrombose artérielle, il est particulièrement avantageux de chercher à prévenir la resténose artérielle, pathologie qui, lorsqu'elle se déclare, peut aboutir à une thrombose de l'artère.

Le polysaccharide sulfaté utilisé dans la présente invention a avantageusement une masse molaire inférieure à 5 000 g/mol.

Selon un autre mode de réalisation avantageux de la présente invention, ledit médicament est destiné à être administré par voie parentérale, de préférence par voie intraveineuse ou sous-cutanée.

Chez le lapin, dans un modèle de thrombose veineuse expérimentale, après injection par voie sous-cutanée, une même activité antithrombotique est observée pour des doses d'HBPM et de polysaccharide sulfaté (polysaccharide susceptible d'être obtenu par dépolymérisation radicalaire d'un fucane brut issu de Phéophycées, comme décrit ci-dessus) respectivement égales à 1 mg/kg et à 10 mg/kg, c'est-à-dire pour une dose de polysaccharide sulfaté 10 fois supérieure à celle d'une HPBM.

Si une extrapolation chez l'homme est possible, les dose journalières dudit médicament sont de préférence comprises entre 150 et 300 mg (administration de façon préventive) ou entre 450 et 600 mg (administration de façon curative) par voie sous-cutanée ; il est bien entendu toutefois que l'Homme de l'Art adaptera ces doses en fonction de l'âge, du poids et de la pathologie du patient, notamment en fonction du risque thrombogène.

Dans le mode de mise en oeuvre relatif à la prévention de la resténose artérielle, ledit médicament est avantageusement destiné à être administré par voie locale, par exemple par diffusion endopariétale.

La diffusion endopariétale consiste en une administration locale de la composition pharmaceutique, par exemple au moyen d'un ballonnet. Le passage répété du ballonnet, lors de l'intervention chirurgicale d'angioplastie, a pour effet de supprimer l'endothélium et de stresser les cellules musculaires et la matrice extracellulaire de la *media*. En réponse à la désendothélialisation, une agrégation plaquettaire va se reproduire au site de la lésion, libérant localement du PDGF (Platelet Derived Growth Factor). En réponse au stress médial, le FGF intracellulaire stocké dans la matrice va être relargué. Ces facteurs de croissance vont induire l'activation, la migration et la prolifération des cellules musculaires lisses.

La technique de diffusion endopariétale permet une distribution topique endovasculaire du principe actif présent dans ladite composition pharmaceutique. Différents types de ballons peuvent être utilisés, tels que des cathéters à doubles ballonnets isolant une chambre de perfusion ou des ballonnets enduits de gel, ou encore des cathéters non occlusifs.

Outre les dispositions qui précèdent, l'invention comprend encore d'autres dispositions qui ressortiront de la description qui va suivre, qui se réfère à des exemples de mesure de l'activité antithrombotique, du risque hémorragique et de l'effet anticoagulant de polysaccharides sulfatés obtenus par dépolymérisation d'un fucane de Phéophycées, ainsi qu'à l'étude des effets de ces polysaccharides sur l'agrégation plaquettaire.

Il doit être bien entendu toutefois que ces exemples sont donnés uniquement à titre d'illustration de l'objet de l'invention, dont ils ne constituent en aucune manière une limitation.

### EXEMPLE 1 : ACTIVITE ANTITHROMBOTIQUE DE POLYSACCHARIDES SULFATES OBTENUS PAR DEPOLYMERISATION D'UN FUCANE DE PHEOPHYCEES

### 1. Préparation du fucane de bas poids moléculaire et de ses références

Dans cet exemple et dans ceux qui suivent, sauf indication contraire, les références du fucane de bas poids_moléculaire sont les suivantes :
- l'héparine standard non fractionnée (HNF) est une héparine TERHORMON TH/023 (150 UI/mg) fournie par Terdobliate (Novara, Italie). Sa masse moléculaire moyenne est d'environ 15 000 g/mol ;
- l'héparine de bas poids moléculaire (HBPM) est fournie par Pharmacia (France) sous la dénomination Fragmine^{®} (lot 94134, 2 500 UI anti-Xa/0,2 ml). Sa masse moléculaire moyenne est d'environ 5 000 g/mol.

Les concentrations requises en HNF et en HBPM sont obtenues par dilution avec du sérum physiologique.

Le fucane de bas poids moléculaire (FBPM) utilisé est obtenu par dépolymérisation radicalaire d'un fucane de Phéophycées (*Ascophyllum nodosum*) conformément au procédé décrit dans la Demande de Brevet EP 0 846 129. Le protocole mis en oeuvre est le suivant :

### - Dépolymérisation radicalaire.

25 litres d'extrait aqueux de fucane, obtenu selon le protocole décrit par Nishino et al. (Carbohydrate Research, 1989, 186, 119-129) adapté à l'algue *Ascophyllum nodosum,* sont introduits dans un réacteur d'une capacité de 45 litres muni d'un dispositif d'agitation à 100 tours/minutes. La température est portée et maintenue à 60°C. 75 g d'acétate de cuivre (Fluka) sont ajoutés, soit une concentration de 0,02 M en acétate de cuivre. Le pH est ajusté à 7,5 à l'aide d'environ 400 ml de soude 2 N (NaOH à 400 g/l, Panréac). On ajoute alors du peroxyde d'hydrogène à une concentration de 10 à 13% à un débit de 85 ml/minutes, pendant 4 h.

### - Elimination du cuivre.

Le milieu réactionnel est filtré sur des filtres en microfibres de verre dont le diamètre des pores est de 2,6 µm (filtres Wathman de référence GF/D 1823-150), afin d'éliminer le précipité de couleur verte formé lors de la réaction de dépolymérisation. Le cuivre résiduel est ensuite retenu par un passage sur une résine (Chelex^{®} 20, Biorad) : l'extrait dépolymérisé est introduit, à un débit de 13 à 15 l/h, dans une colonne de verre d'une section de 113 cm² contenant 5 litres de résine préalablement passivée. En sortie de colonne, la solution de fucane dépolymérisé est décolorée et a un pH compris entre 10 et 11.

La résine utilisée est ensuite régénérée selon les conditions opératoires données par le fabricant.

### - Diafiltration, concentration et lyophilisation.

La solution de fucane dépolymérisé obtenue ci-dessus est soumise à une ultrafiltration sur un système Pellicon^{®} (Millipore) équipé de deux membranes polysulfones de 1 kDalton (Filtron). Un suivi de conductivité est effectué tout au long de ce procédé, réalisé avec 10 volumes d'eau osmosée.

Après concentration jusqu'à un volume final de 4 à 5 litres, le produit est lyophilisé. Le rendement, calculé sur le lyophilisat, est de 27%.

### - Réduction au borohydrure.

267 g du fucane dépolymérisé obtenu ci-dessus (fucane lyophilisé) sont dissous dans 3 1 d'eau osmosée et la solution est homogénéisée. 10 ml de cette solution sont prélevés pour analyse du fucane avant l'étape de réduction des monosaccharides terminaux des chaînes polysaccharidiques.

Séparément, 202 g de borohydrure de sodium sont dissous dans 3 1 d'eau. La solution de borohydrure est ensuite ajoutée à la solution de fucane. La réaction est immédiate et très effervescente. Après 2 heures, la réaction est stoppée par addition d'acide acétique 10 N (acide acétique glacial, Panréac) jusqu'à l'obtention d'un pH neutre. Le volume d'acide ajouté est de 400 ml.

Après neutralisation, la solution est filtrée sur microfibres de verre (fitre Wathman de référence GF/D 1822-290) et soumise à une ultrafiltration/diafiltration sur un système Pellicon^{®} (Millipore) équipé de deux membranes polysulfones de seuil de coupure de 1 kDalton (Filtron). Le volume initial est de 9 1 ; la conductivité initiale est de 16,5 mS et chute à 3,5 mS en fin d'ultrafiltration/diafiltration. Après une concentration jusqu'à 7,5 1, le produit est filtré successivement sur des filtres de diamètres de pores de 2,6 µm (GF/D Wathman), puis 0,7 µm (GF/F Wathman) et enfin 0,2 µm (filtres Wathman en nitrate de cellulose, référence 7182-09). Le volume final à lyophiliser est de 7,75 1. Le rendement global de la réduction est de 60%, cette faible valeur étant due aux nombreuses manipulations réalisées lors de cette étape.

Le rendement total du procédé d'obtention d'un fucane de bas poids moléculaire (dépolymérisation et réduction) est par conséquent de 16,2%.

L'étape de réduction ne modifie pas l'activité anticoagulante et la composition chimique du fucane, comme cela sera détaillé ci-après (cf. tableau I).

Le tableau I résume les caractéristiques du produit (fucane dépolymérisé) avant et après l'étape de réduction, à savoir sa masse molaire (Mc : masse molaire chromatographique déterminée au sommet du pic), son indice de polydispersité (correspondant au rapport Mₚ/Mₙ, avec Mₚ : masse moléculaire moyenne en poids et Mₙ : masse moléculaire moyenne en nombre), sa teneur en oses neutres (fucose, galactose, xylose), en acide uronique, en sulfate (SO₃Na), ainsi que la mesure de l'activité anticoagulante du produit par TCA (Temps de Céphaline Activée ), exprimée en quantité de produit ajoutée à 1 ml de PPP (Plasma Pauvre en Plaquettes) nécessaire pour doubler le temps de coagulation témoin (39 secondes).

La teneur en oses neutres a été mesurée selon la technique décrite par Mopper et al. (environ. Sci. Technol., 1992, 26, n°1, 133-138). Les autres paramètres ont été mesuré comme indiqué dans la Demande de Brevet EP 0 846 129.

Il ressort du tableau I que le procédé de réduction décrit ci-dessus, réalisé après la dépolymérisation radicalaire, permet d'obtenir un fucane de bas poids moléculaire peu polydisperse, sans modifier globalement sa composition chimique et sans le désulfater.

**Tableau I**

| | Fucane avant dépolymérisation (extrait aqueux) | Fucane dépolymérisé, avant réduction | Fucane dépolymérisé, après réduction |
|---|---|---|---|
| Mc (g/mol) | > 600 000 | < 5000 | < 5000 |
| indice de polydispersité | 5 | 1,9 | 1,7 |
| fucose (%) | 28,4 | 32,8 | 34,3 |
| galactose (%) | 3,4 | 1,6 | 1,4 |
| xylose (%) | 3,7 | 1,2 | 1,0 |
| acide uronique (%) | 8,5 | 3,7 | 2,7 |
| SO₃Na (%) | 22,7 | 34,2 | 32,2 |
| TCA (activité anticoagulante) (µg/ml de PPP) | nd* | 25 | 25 |

| | | | |
|---|---|---|---|
| * nd : non déterminé | | | |

### 2. Thrombose veineuse selon Wessler chez le lapin

### Protocole

La thrombose veineuse expérimentale est celle de Wessler *et al*. (1959) utilisant le facteur X activé (FXa) comme agent hypercoagulant (Mauray et al., Throm. Haemost., 1995, 74, 1280-5 et Millet et al., Throm. Haemost., 1992, 67, 176-9).

Comme décrit par Millet et al. (Throm. Haemost, 1999, 81, 391-395), les produits sont administrés par voie sous-cutanée, un effet dose et une cinétique de l'effet étant réalisés.

### Résultats

Le FBPM présente une activité dose dépendante qui se manifeste dès la dose de 2,5 mg/kg, avec un abaissement significatif et voisin de 35% du poids des thrombi. Cette activité est proche de 100% pour la dose de 10 mg/kg.

Des activités de 50%, 80% et 90% ont été obtenues pour des doses respectives de Fragmine® de 80, 100 et 200 UI anti-Xa/kg (Millet *et al*., 1999, *ibid*).

Le calcul mathématique, utilisant une régression logarithmique, permet la détermination de la dose de FBPM nécessaire pour diminuer de 80% (DE 80) le poids des thrombi témoins. Ainsi, la DE 80 du FBPM, par vois sous-cutanée, est de 7,4 (5,6-9,8) mg/kg.

Comme observé par Millet *et al*., 1999 (*ibid*) sur le fucane de bas poids moléculaire obtenu par hydrolyse acide et en prenant la Fragmine^{®} comme produit de référence, le FBPM montre, chez le lapin, une cinétique de l'activité antithrombotique plus précoce et plus durable.

### 3. Thrombose artério-veineuse selon Umetsu chez le lapin

### 3-a : activité antithrombotique en intraveineux

### • Protocole

Les animaux utilisés sont des lapins mâles néo-zélandais homozygotes de 2 à 2,5 kg de poids moyen. Après une stabulation des animaux durant 6 jours en animalerie, ceux-ci sont utilisés en période de jeûne.

Après prémédication des animaux à l'Imalgène^{®}, ceux-ci sont anesthésiés par une injection intraveineuse de pentobarbital sodique à 1% dans la veine marginale de l'oreille. L'injection se fait lentement avec un contrôle du réflexe pupillaire et de la respiration. Environ 4 à 5 ml de pentobarbital sont injectés à un lapin de 2 kg.

Une artère carotide et une veine jugulaire opposée sont dégagées. Un shunt artério-veineux est placé entre ces deux vaisseaux. Ce shunt est composé de 2 cathéters en polyéthylène (diamètre extérieur de 1,9 mm) de 12,5 cm de longueur, ces deux cathéters étant reliés entre eux par un troisième cathéter d'un diamètre intérieur de 1,57 mm et d'une longueur de 6 cm, à l'intérieur duquel est placé un fil de soie.

Les cathéters sont siliconés (Sigmacote^{®}, Sigma). Une bague Doppler de 1,3 mm de diamètre est fixée autour du cathéter introduit dans la carotide afin d'apprécier la variation de vélocité du flux sanguin, représentative de l'occlusion.

Un premier groupe de lapins, qui servira de contrôle, reçoit du sérum physiologique, trois autres groupes recevant respectivement une solution d'HNF (1 mg/kg) et deux solutions de FBPM (1 mg/kg et 2 mg/kg). Les diverses solutions et leurs solvants (le sérum physiologique) sont administrées sous un volume de 1 ml/kg, par injection intraveineuse dans une veine marginale de l'oreille (opposée à celle servant au maintien de l'anesthésie de l'animal), 5 minutes avant la création de la situation thrombogène.

### • Résultats

Le tableau II résume les temps d'occlusion (en minutes) en fonction des doses de FBPM et d'HNF administrés par injection intraveineuse. Le symbole *** indique le seuil de significativité d'une probabilité égale ou inférieure à 0,1% (p ≤ 0,001 dans le test statistique de Student).

**Tableau II**

| | Témoin | HNF 1 mg/kg | FBPM | |
|---|---|---|---|---|
| | | | 1 mg/kg | 2 mg/kg |
| Temps d'occlusion (min.) | 15,6 ± 1,7 | 55,4 ± 2,9^{***} | 27,6 ± 6,5 | 43,7 ± 6,0^{***} |
| Nombre d'animaux | 9 | 5 | 7 | 6 |

Il ressort de ces résultats que le FBPM, administré par voie intraveineuse dans le modèle de Umetsu adapté au lapin, présente une activité antithrombotique artérielle à des doses proches de celles responsables d'une activité antithrombotique veineuse.

Un doublement du temps d'occlusion est noté 5 minutes après une administration par voie intraveineuse de FBPM à la dose de 1 mg/kg (dose voisine de la DE 80 antithrombotique veineuse). L'HNF administrée à la dose de 1 mg/kg, dans les mêmes conditions, augmente de près de 4 fois le temps nécessaire pour l'obtention d'une obstruction vasculaire totale chez les animaux témoins. Par ailleurs, il faut noter que cette dose correspond à 10 fois la DE 80 antithrombotique veineuse de l'HNF.

### 3-b : activité antithrombotique en sous-cutané

### • Protocole

On procède comme indiqué ci-dessus, les diverses solutions et leurs solvants (le sérum physiologique) étant administrées sous un volume de 1 ml/kg, par voie sous-cutanée, 2 heures avant la création de la situation thrombogène. Les animaux sont répartis en trois groupes : un premier groupe, qui servira de contrôle, reçoit du sérum physiologique, alors que les autres groupes reçoivent respectivement des solutions de FBPM à la dose de 7,5 mg/kg et d'HBPM à la dose de 60 UI anti-Xa/kg.

### • Résultats

Le tableau III résume les temps d'occlusion (en minutes) en fonction des doses de FBPM et d'HBPM administrés par injection sous-cutanée.

**Tableau III**

| | Témoin | HBPM (60 UI anti-Xa/kg) | FBPM (7,5 mg/kg) |
|---|---|---|---|
| Temps d'occlusion (min.) | 12,6 ± 1,0 | 11,8 ± 1,3 | 22,8 ± 3,4 |
| Nombre d'animaux | 5 | 4 | 4 |

Il ressort de ces résultats qu'aux doses testées en sous-cutané, le FBPM double le temps d'occlusion témoin, alors que l'HBPM n'a aucun effet.

Contrairement à ce qui est noté pour l'HNF (en injection intraveineuse) et pour l'HBPM (injection sous-cutanée), la dose de FBPM nécessaire pour doubler le temps d'occlusion artériel est très proche de la DE 80 antithrombotique veineuse (à savoir 7,4 mg/kg), à la différence des héparines, pour lesquelles il est nécessaire de multiplier par un facteur voisin de 10 la dose antithrombotique veineuse pour obtenir une dose active en thrombose artérielle.

### 4. Thrombose artérielle au FeCl₃ chez le rat

### • Protocole

Seule l'administration par voie intraveineuse a été étudiée dans ce modèle.

Les animaux utilisés sont des rats mâles Wistar de poids variant de 240 à 390 g. Après une stabulation des animaux durant 6 jours en animalerie, ceux-ci sont utilisés en période de jeûne.

Après une anesthésie au carbonate d'éthyle à 15% (10 ml/kg ; Prolabo, Paris, France), une artère carotide est exposée, soigneusement dégagée des tissus ou des nerfs environnants et un carré de parafilm est placé sous la carotide. Une sonde Doppler est placée autour de la carotide (côté céphalique). Le signal Doppler est ajusté de manière à avoir une déflexion maximale et assimilée à une valeur de 100%.

Après une période de stabilisation du flux sanguin, un disque (3 mm de diamètre) de papier filtre Wathman est placé sur la carotide de manière distale par rapport à la sonde. Deux microlitres d'une solution de FeCl₃ (Osi, Paris, France) à 25% dans de l'acide chlorhydrique 1 M sont déposés sur le papier filtre, qui est alors placé à la verticale du parafilm au contact de la carotide dégagée. Un chronomètre est déclenché. Après 30 secondes de contact du papier filtre, celui-ci est enlevé. La formation du thrombus est alors suivie par l'analyse du signal Doppler.

L'évaluation de la thrombose, exprimée en minutes, consiste à mesurer l'intervalle de temps séparant l'application du FeCl₃ du moment où le signal Doppler rejoint la ligne de base, traduisant ainsi une occlusion totale.

Les animaux sont répartis en trois principaux groupes, l'un recevant le sérum physiologique (groupe de contrôle), et les autres recevant respectivement le FBPM (à des doses variant de 1,25 à 10 mg/kg) et l'HNF (aux doses de 1,25 et 2,5 mg/kg). Le sérum physiologique, ainsi que les solutions de FBPM et d'HNF, sont injectées par voie intraveineuse, sous un volume de 1 ml/kg, 5 minutes avant l'induction de la thrombose.

### • Résultats

Le tableau IV résume les temps d'occlusion artérielle (en minutes), ainsi que le facteur de multiplication du temps d'occlusion (calculé par rapport au temps d'occlusion témoin), en fonction des doses de FBPM et d'HNF administrés par voie intraveineuse. Les symboles ^{***} et ^{**} indiquent les seuils de significativité d'une probabilité respectivement égale ou inférieure à 0,1% et 1% (respectivement, p ≤ 0,001 et p ≤ 0,01 dans le test statistique de Student).

**Tableau IV**

| | temps d'occlusion (min.) | facteur de multiplication | nombre d'animaux |
|---|---|---|---|
| témoin | 11,6 ± 0,58 | / | 19 |

| FBPM : | | | |
|---|---|---|---|
| 10 mg/kg | 52,5 ± 7,5^{***} | 4,52 | 6 |
| 5 mg/kg | 41,7 ± 8,9^{***} | 3,59 | 7 |
| 2,5 mg/kg | 31,9 ± 9,3^{**} | 2,75 | 6 |
| 1,25 mg/kg | 12,7 ± 1,4 | 1,09 | 5 |

| HNF : | | | |
|---|---|---|---|
| 2,5 mg/kg | 60,0 ± 0^{***} | 5,17 | 2 |
| 1,25 mg/kg | 20,5 ± 0,5^{**} | 1,76 | 3 |

Comme le montre le tableau ci-dessus, le temps d'occlusion est de 20,5 minutes après une injection d'HNF à la dose de 1,25 mg/kg et est de 60 minutes lorsque l'HNF est administrée à la dose de 2,5 mg/kg, soit plus de 5 fois le temps témoin.

Quant au FBPM, il présente une activité significative dose dépendante. Dès la dose de 2,5 mg/kg, l'injection de FBPM induit un retard significatif du temps d'occlusion artériel, temps qui apparaît plus que doublé par rapport au temps témoin.

Un calcul mathématique (régression linéaire) permet de déduire des résultats ci-dessus les doses de FBPM et d'HNF nécessaires pour doubler le temps d'occlusion artérielle, qui sont voisines de 2 mg/kg pour le FBPM et de 1,25 mg/kg pour l'HNF.

### EXEMPLE 2 : RISQUE HEMORRAGIQUE ET EFFET ANTICOAGULANT DE POLYSACCHARIDES SULFATES OBTENUS PAR DEPOLYMERISATION D'UN FUCANE DE PHEOPHYCEES

### 1. Risque hémorragique chez le lapin

### a) Matériel et méthode

Chez le lapin, le risque hémorragique du FBPM et de l'HBPM est évalué par la mesure de l'allongement des temps de saignement, selon le modèle de Carter *et al*. modifié par Doutremepuich et al. (Throm. Res., 1979, 15, 581-586), après administration des produits par voie sous-cutanée, 2 heures avant l'induction de l'hémorragie, à des doses proches de la DE 80 antithrombotique veineuse et de 5 fois cette dose.

Le fucane de bas poids moléculaire (FBPM) et les animaux utilisés sont identiques à ceux décrits dans l'exemple 1. Dans le cas de l'injection à 1 x DE 80, l'héparine de bas poids moléculaire (HBPM) est la Fragmine^{®} telle que décrite dans l'exemple 1 ; dans le cas de l'injection à 5 x DE 80, il s'agit de la Tinzaparine^{®} (10 000 UI anti-Xa/0,5 ml ; lot Q 6369B), fournie par la Société Innohep (France).

### b) Résultats

A la dose permettant de doubler le temps témoin d'occlusion artérielle, correspondant à la DE 80 antithrombotique veineuse, le FBPM et l'HBPM administrés par voie sous-cutanée n'ont pas d'activité hémorragipare. On note également qu'à cette dose, et à la différence du FBPM, l'HBPM est inactive chez le lapin dans le modèle de thrombose artérielle selon le modèle d'Umetsu.

A 5 fois cette dose, ni l'HBPM, ni le FBPM n'induisent une augmentation significative du temps de saignement.

### 2. Risque hémorragique chez le rat

### a) Matériel et méthodes

Chez le rat, le risque hémorragique du FBPM, de l'HNF et de l'HBPM est évalué par la mesure de l'allongement des temps de saignement, selon le modèle de Dejana et al. (Throm. Haemost., 1982, 48, 108-111). Le FBPM, l'HNF et les animaux utilisés sont identiques à ceux décrits dans l'exemple 1.

Les composés sont administrés par voie intraveineuse à des doses-de 5 et 10 mg/kg pour le FBPM (doses correspondant respectivement à 2,5 fois et à 5 fois la dose doublant le temps d'occlusion (TO) témoin (cf. tableau IV), de 750 et 1 500 UI anti-Xa/kg pour l'HBPM (Fragmine^{®}) et de 1 mg/kg pour l'HNF, 5 minutes avant l'induction de l'hémorragie.

### b) Résultats

Le tableau V résume, en pourcentages, l'allongement des temps de saignement obtenus.

**Tableau V**

| doses | produits | allongement des temps de saignement | nombre d'animaux |
|---|---|---|---|
| 1 X TO x 2 | FBPM | nd^{*} | |
| | HNF | >80% | 9 |
| 2,5 X TO x 2 | FBPM | >50% | 8 |
| | HBPM | >200% | 8 |
| 5 X TO x 2 | FBPM | >200% | 3 |
| | HBPM | >200% | 3 |

| | | | |
|---|---|---|---|
| * nd : non déterminé | | | |

Il ressort de ce tableau qu'à 2,5 fois la dose nécessaire pour doubler le temps d'occlusion témoin, le FBPM augmente bien moins le temps de saignement que l'HBPM. A 5 fois cette dose, les allongements des temps de saignement sont similaires pour les deux produits.

Ainsi, en comparaison à une HNF et à une HBPM; il ressort du tableau V que le FBPM présente un risque hémorragique modéré.

### 3. Effet anticoagulant ex vivo

### a) Matériel et méthode

On utilise les plasmas provenant :
- de lapins dans lesquels une thrombose veineuse selon le modèle de Wessler a été induite ;
- des rats utilisés dans l'expérience de thrombose artérielle selon l'exemple 1, ces rats ayant reçu, par voie intraveineuse, des doses proches de celle nécessaire pour doubler le temps d'occlusion témoin, soit 2,5 mg/kg de FBPM ou 1,25 mg/kg d'HNF, ou des doses doubles, c'est-à-dire 5 mg/kg de FBPM ou 2,5 mg/kg d'HNF.

L'injection d'une solution saline physiologique sert de contrôle. Le plasma des animaux est prélevé juste après la formation du sac veineux.

La mesure.du temps de céphaline activé (TCK ou TCA) est effectuée comme suit :
- pour les plasmas prélevés chez les lapins, 100 µl de plasma et 100 µl de réactif CKPrest (Stago, Asnières, France) sont incubés pendant 3 minutes à 37°C, puis on ajoute 100 µl de CaCl₂ pour déclencher la coagulation. On obtient ainsi une mesure du TCK (Temps de Céphaline Kaolin) ;
- pour les plasmas prélevés chez les rats, le TCA est mesuré sur l'automate ACL 3000 (Delhomme, Paris, France). 53 µl de plasma et 53 µl de céphaline activée par de l'acide ellagique (fourni par IL, Paris, France, sous la référence 97570-10) sont mis en contact, après incubation à 37°C. La coagulation est déclenchée par l'adjonction de 53 µl de CaCl₂. L'augmentation de la turbidité due à la formation du caillot fibrineux est détectée par néphélométrie.

La mesure du temps de thrombine (TT) est effectuée comme suit :
- pour les plasmas prélevés chez les lapins, on met en présence 100 µl de thrombine (thrombine humaine fournie par Fibrindex Ortho Diagnostic Système, Roissy, France) avec 200 µl de plasma, après incubation pendant 2 minutes à 37°C. L'addition de la thrombine au plasma déclenche le processus de formation du caillot de fibrine ;
- pour les plasmas prélevés chez les rats, le TT est mesuré sur l'automate ACL 3000 décrit ci-dessus. Le plasma et la thrombine humaine (référence 0880, Stago, Asnières, France), après incubation à 37°C, sont mis en contact volume à volume (75 µl). La formation d'un caillot de fibrine augmente la turbidité, qui est détectée par néphélométrie sur l'automate de coagulation.

### b) Résultats

### • Effet anticoagulant chez le rat (administration des composés par voie intraveineuse).

Chez le rat, l'injection intraveineuse de FBPM prolonge le TCA et le TT de façon significative, mais de façon bien inférieure à ce qui est observé avec l'HNF.

### • Effet anticoagulant chez le lapin (administration des composés par voie sous-cutanée).

Les résultats des TCK obtenus chez le lapin, exprimés en secondes, sont résumés dans le tableau VI ci-dessous. Les symboles ^{*} et ^{**} indiquent les seuils de significativité d'une probabilité respectivement égale ou inférieure à 5% et 1% (respectivement, p ≤ 0,05 et p ≤ 0,01 dans le test statistique de Student).

**Tableau VI**

| | | | |
|---|---|---|---|
| | dose | TCK (secondes) | nombre d'animaux |
| contrôle | 0 | 25,1 ± 1,3 | 8 |
| FBPM | 7,5 mg/kg | 28,2 ± 1,8 | 6 |
| | 10 mg/kg | 29,4 ± 1,5* | 7 |
| HBPM | 50 UI anti-Xa/kg | 25,7 ± 1,7 | 3 |
| | 100 UI anti-Xa/kg | 27,0 ± 1,5 | 3 |
| | 200 UI anti-Xa/kg | 33,9 ± 5,0** | 3 |

Il ressort du tableau VI que l'HBPM (Fragmine^{®}), administrée à des doses allant de 50 à 200 UI anti-Xa/kg, 2 heures avant les prélèvements sanguins, n'induit qu'à une très forte dose (200 UI anti-Xa/kg) une élévation significative du TCK (35,3% d'augmentation par rapport au groupe de contrôle).

Le TCK ne subit aucune modification significative pour la dose de FBPM de 7,5 mg/kg, administrée par voie sous-cutanée 2 heures avant les prélèvements sanguins. A la dose de 10 mg/kg, on note une augmentation discrète, mais significative (17% d'augmentation).

Les résultats des TT obtenus chez le lapin, exprimés en secondes, sont résumés dans le tableau VII ci-dessous. Le symbole ^{**} indique le seuil de significativité d'une probabilité égale ou inférieure à 1% (p ≤ 0,01 dans le test statistique de Student).

**Tableau VII**

| | dose | TT (secondes) | nombre d'animaux |
|---|---|---|---|
| contrôle | 0 | 31,6 ± 1,3 | 9 |
| FBPM | 7,5 mg/kg | 34,7 ± 2,2 | 6 |
| | 10 mg/kg | 31,5 ± 1,2 | 7 |
| HBPM | 50 UI anti-Xa/kg | 32,5 ± 2,7 | 3 |
| | 100 UI anti-Xa/kg | 38,2 ± 4,1 | 3 |
| | 200 UI anti-Xa/kg | > 98^{**} | 3 |

Il ressort du tableau VII que le temps de thrombine moyen (TT) ne subit aucune modification significative pour les doses de FBPM testées, administrées par voie sous-cutanée 2 heures avant les prélèvements sanguins. On note que l'HBPM (Fragmine^{®}), aux doses testées, augmente le TT.

### EXEMPLE 3 : ETUDE EX VIVO DE L'EFFET DE POLYSACCHARIDES SULFATES OBTENUS PAR DEPOLYMERISATION RADICALAIRE D'UN FUCANE DE PHEOPHYCEES SUR l'AGREGATION PLAQUETTAIRE

### a) Matériel et méthode

Le FBPM et l'HNF sont tels que décrits dans l'exemple 1. L'HBPM est de la Tinzaparine^{®}, fournie par Innohep.

### • Chez le lapin.

Le FBPM et l'HBPM sont injectés, par voie sous-cutanée, à dose voisine de 5 x DE 80 (5 fois la dose nécessaire pour diminuer de 80% le poids moyen des thrombi témoins) déterminée selon le modèle de thrombose veineuse selon Wessler (DE 80 de la Tinzaparine^{®} par injection sous-cutanée : 80 UI anti-Xa/kg). Ainsi, le FBPM et l'HBPM sont injectés, aux doses respectives de 37,5 mg/kg et de 400 UI anti-Xa/kg. Quant à l'HNF, elle est administrée par voie intraveineuse à une dose voisine de 10 x DE 80, c'est-à-dire à raison de 1 mg/kg.

### - Agrégation à la thrombine.

De la thrombine bovine à 100 U NIH (National Institute of Health)/ml, (Sigma, référence 4648), conservée à -70°C, est décongelée et diluée au 1/10 dans du sérum physiologique, puis conservée dans de la glace pendant la durée de l'expérimentation. A 300 µl de PRP (Plasma Riche en Plaquettes), obtenu par centrifugation du sang des animaux pendant 10 minutes à 400 g , et pré-incubés pendant 1 minute à 37°C, sont ajoutés 100 µl de tampon PBS et une quantité de thrombine (quantité voisine de 8 µl) nécessaire pour avoir une concentration finale proche de 0,2 U NIH/ml.

### - Agrégation à l'ADP (adénosine diphosphate).

Une solution d'ADP à 100 µM (référence 0494, Stago, Asnières, France), conservée à -20°C, est décongelée, diluée au 1/25 et 1/12,5 dans du tyrode calcique (NaCl 138,6 mM ; KCl 2,8 mM ; NaHCO₃ 11,9 mM ; MgCl₂ 1,1 mM ; NaH₂PO₄ 0,33 mM ; glucose 11,2 mM ; CaCl₂ 4 µM ; Laboratoires FOURNIER) et mise dans de la glace pendant la durée de l'expérimentation. Après incubation de 300 µl de PRP à 37°C pendant 1 minute, l'agrégation plaquettaire est déclenchée par 100 µl d'une solution d'ADP (concentration finale de 1 et 2 µM).

### • Chez le rat.

Le FBPM et l'HNF sont injectés par voie intraveineuse, 5 minutes avant les prélèvements sanguins, aux doses respectives de 2,5 et 5 mg/kg pour le FBPM et de 1,25 et 2,5 mg/kg pour l'HNF.

Les protocoles d'agrégation plaquettaire sont similaires à ceux décrits ci-dessus, aux différences près suivantes pour le protocole d'agrégation à la thrombine : le tampon ajouté au PRP est un tampon tyrode recalcifié, et la concentration finale en thrombine est de 0,6 U NIH/ml.

### b) Résultats

### • Chez le lapin.

Le FBPM et l'HBPM (à la dose de 5 x DE 80) et l'HNF (à la dose de 10 x DE 80) inhibent à 100% l'agrégation plaquettaire induite par la thrombine, mais n'inhibent pas l'agrégation plaquettaire induite par 1 µM et 2 µM d'ADP.

### • Chez le rat.

Le FBPM et l'HNF inhibent à 100% l'agrégation plaquettaire induite par la thrombine.

A l'inverse de ce qui est noté suite à l'injection d'HNF (absence d'inhibition ou très faible inhibition de l'agrégation plaquettaire), le FBPM, quelle que soit la dose injectée, réduit de 35 à 45% l'agrégation plaquettaire induite par l'ADP, sans qu'un effet dose puisse être noté.

### EXEMPLE 4 : ETUDE IN VIVO DE L'EFFET DE POLYSACCHARIDES SULFATES OBTENUS PAR DEPOLYMERISATION RADICALAIRE D'UN FUCANE DE PHEOPHYCEES SUR LA PREVENTION DE LA RESTENOSE ARTERIELLE.

Le traitement des sténoses artérielles par voie endovasculaire est une technique couramment pratiquée. Elle consiste à introduire dans l'artère un ballonnet que l'on vient gonfler en regard de la sténose (opération d'angioplastie) dans le but de restaurer un calibre vasculaire normal. L'angioplastie est le plus souvent suivie de la pose d'un stent qui permet, grâce à sa force d'expansion radiale, de maintenir le calibre de l'artère. Cette technique est fréquemment utilisée dans le traitement des sténoses athéromateuses coronaires, iliaques ou rénales. Le traumatisme pariétal induit par l'angioplastie et la pose de stent est malheureusement responsable d'une resténose du vaisseau dans 20 à 30% des cas chez l'homme. Cette resténose est en grande partie due à une prolifération et une migration des cellules musculaires lisses (CML) de la *media* vers l'intima du vaisseau. L'apparition d'une hyperplasie néointimale réduit le calibre du stent, favorise l'ischémie d'aval et est source, chez l'homme, d'une morbidité et d'une mortalité non négligeables.

Des fractions de polysaccharide sulfaté susceptibles d'être obtenues par dépolymérisation radicalaire d'un fucane brut issu de Phéophycées, ledit polysaccharide ayant une masse molaire inférieure ou égale à 10 000 g/mol, peuvent être utilisées, conformément à l'invention, pour prévenir la resténose artérielle, comme démontré ci-dessous dans un modèle de resténose chez le lapin, dans l'artère iliaque.

### a) Matériel et méthode

Les animaux sont des lapins New Zealand d'un poids moyen de 3,5 à 4 kg. Ils sont anesthésiés au pentobarbital.

10 stents iliaques sont implantés chez 5 lapins. Un stent est positionné dans chaque artère iliaque (abord carotidien). La pose de chaque stent est précédée de 3 angioplasties d'une minute à 10 atmosphères (atm). Le stent est posé sous une pression de 10 atm pendant 30 secondes.

Les animaux sont ensuite traités pendant 14 jours avec le FBPM obtenu dans l'exemple 1, injecté 2 fois par jour en intramusculaire à la dose de 10-mg/kg/24 h. Le groupe témoin est constitué de 6 artères stentées chez 3 lapins.

Après 14 jours, les animaux sont sacrifiés. On prélève l'aorte abdominale basse et les artères iliaques, qui sont fixées sous pression (dans le formol) et incluses dans du métacrylate. On effectue ensuite des coupes au microtome afin de réaliser des analyses histologiques et histomorphométriques, et de calculer la surface des artères.

### b) Résultats

Les mesures de surface réalisées (*intima, media*, rapports *intima*/*media* et *intima*/*limitante* élastique interne), rassemblées dans le tableau VIII (dans lequel « n » représente le nombre de mesures effectuées), indiquent une diminution de l'hyperplasie intimale dans le groupe d'animaux traités par le FBPM. Cette diminution de l'hyperplasie intimale est très importante (de l'ordre de 60%).

Il est également à noter qu'en plus de la diminution de la surface de l'*intima* et des rapports *intima*/*media* et *intima*/limitante élastique interne, le fucane n'a pas d'effet sur la surface de la *media,* ce qui révèle une inhibition spécifique de la prolifération des CML.

**Tableau VIII**

| | Témoins (n = 6) | Animaux traités par le FBPM (n = 10) | Diminution (%) |
|---|---|---|---|
| **intima** | 1,83 | 0,73 | 60 |
| e type | 0,51 | 0,20 | |
| ***media*** | 0,41 | 0,37 | n.s. |
| e type | 0,02 | 0,19 | |
| ***intima*/*média*** | 4,48 | 1,97 | 56 |
| e type | 1,47 | 0,65 | |
| ***intima*/LEI** | 0,35 | 0,17 | 52 |
| e type | 0,08 | 0,06 | |

| | | | |
|---|---|---|---|
| e type = écart type, n.s. = non significatif LEI = limitante élastique interne | | | |

## Revendications

1. Utilisation d'un polysaccharide sulfaté susceptible d'être obtenu par dépolymérisation radicalaire d'un fucane brut issu de Phéophycées, ledit polysaccharide ayant une masse molaire inférieure ou égale à 10 000 g/mol, pour l'obtention d'un médicament destiné à la prévention ou au traitement de la thrombose vasculaire.

2. Utilisation selon la revendication 1, **caractérisée en ce que** ledit médicament est destiné à la prévention ou au traitement de la thrombose veineuse.

3. Utilisation selon la revendication 1, **caractérisée en ce que** ledit médicament est destiné à la prévention ou au traitement de la thrombose artérielle.

4. Utilisation selon la revendication 3 pour la prévention de la resténose artérielle.

5. Utilisation selon l'une quelconque des revendications 1 à 4, **caractérisée en ce que** ledit polysaccharide sulfaté a une masse molaire inférieure à 5 000 g/mol.

6. Utilisation selon l'une quelconque des revendications 1 à 5, **caractérisée en ce que** ledit médicament est destiné à être administré par voie parentérale.

7. Utilisation selon la revendication 6, **caractérisée en ce que** ledit médicament est destiné à être administré par voie intraveineuse.

8. Utilisation selon la revendication 6, **caractérisée en ce que** ledit médicament est destiné à être administré par voie sous-cutanée.

9. Utilisation selon la revendication 8, **caractérisée en ce que** ledit médicament est destiné à être administré de façon préventive.

10. Utilisation selon la revendication 8, **caractérisée en ce que** ledit médicament est destiné à être administré de façon curative.

11. Utilisation selon la revendication 4, **caractérisée en ce que** ledit médicament est destiné à être administré par voie locale.

12. Utilisation selon la revendication 11, **caractérisée en ce que** ledit médicament est destiné à être administré par diffusion endopariétale.

## Claims

1. Use of a sulfated polysaccharide obtainable by radical depolymerisation of a crude fucane derived from Phaeophyceae, said polysaccharide having a molar mass of less than or equal to 10 000 g/mol, for obtaining a medicament for preventing or treating vascular thrombosis.

2. Use according to claim **1,** wherein said medicament is for the prevention or treatment of venous thrombosis.

3. Use according to claim **1,** wherein said medicament is for preventing or treating arterial thrombosis.

4. Use according to claim **3,** for the prevention of arterial restenosis.

5. Use according to anyone of claims **1** to **4,** wherein said sulphated polysaccharide has a molar mass of less than 5000 g/mol.

6. Use according to anyone of claims 1 to **5,** wherein said medicament is to be administrated parenterally.

7. Use according to claim 6, wherein said medicament is to be administrated intravenously.

8. Use according to claim 6, wherein said medicament is to be administrated subcutaneously.

9. Use according to claim 8, wherein said medicament is to be administrated preventatively.

10. Use according to claim 8, wherein said medicament is to be administrated curatively.

11. Use according to claim 4, wherein said medicament is to be administrated locally.

12. Use according to claim 11, wherein said medicament is to be administrated by endoparietal diffusion.

## Patentansprüche

1. Verwendung eines sulfatierten Polysaccharids, das durch radikalische Depolymerisation aus einem unverarbeiteten Fucan gewonnen werden kann, das aus Phaeophyceae stammt, wobei das Polysaccharid eine molare Masse von unter oder gleich 10.000 g/mol aufweist, zur Herstellung eines Medikaments, das zur Vorbeugung oder für die Behandlung von vaskulären Thrombosen bestimmt ist.

2. Verwendung nach Anspruch 1, **dadurch gekennzeichnet, dass** das Medikament zur Vorbeugung oder für die Behandlung von Venenthrombosen bestimmt ist.

3. Verwendung nach Anspruch 1, **dadurch gekennzeichnet, dass** das Medikament zur Vorbeugung oder für die Behandlung von arteriellen Thrombosen bestimmt ist.

4. Verwendung nach Anspruch 3 zur Vorbeugung von arteriellen Restenosen.

5. Verwendung nach einem der Ansprüche 1 bis 4, **dadurch gekennzeichnet, dass** das sulfatierte Polysaccharid eine molare Masse von unter 5 000 g/mol aufweist.

6. Verwendung nach einem der Ansprüche 1 bis 5, **dadurch gekennzeichnet, dass** das Medikament dafür bestimmt ist, parenteral verabreicht zu werden.

7. Verwendung nach Anspruch 6, **dadurch gekennzeichnet, dass** das Medikament dafür bestimmt ist, intravenös verabreicht zu werden.

8. Verwendung nach Anspruch 6, **dadurch gekennzeichnet, dass** das Medikament dafür bestimmt ist, subkutan verabreicht zu werden.

9. Verwendung nach Anspruch 8, **dadurch gekennzeichnet, dass** das Medikament dafür bestimmt ist, vorbeugend verabreicht zu werden.

10. Verwendung nach Anspruch 8, **dadurch gekennzeichnet, dass** das Medikament dafür bestimmt ist, kurativ verabreicht zu werden.

11. Verwendung nach Anspruch 4, **dadurch gekennzeichnet, dass** das Medikament dafür bestimmt ist, lokal verabreicht zu werden.

12. Verwendung nach Anspruch 11, **dadurch gekennzeichnet, dass** das Medikament dafür bestimmt ist, durch endoparietale Diffusion verabreicht zu werden.
